# EUROPEAN PATENT APPLICATION

(11) **EP 4 697 004 A1**
(43) Date of publication of application: **18.02.2026**
(21) Application number: 24461611.6
(22) Date of filing: 17.08.2024
(51) Int. Cl.: G01N 1/28, G01N 3/00, G01N 33/24

(54) **MOULD FOR PREPARATION OF A SOIL SAMPLE AND A METHOD OF PREPARING A SAMPLE FOR TESTING MECHANICAL PROPERTIES OF SOIL**

(71) Applicant: Instytut Techniki Budowlanej, 00-611 Warszawa (PL)
(72) Inventor: Witowski, Marcin, 05-120 Legionowo (PL)
(74) Representative: AOMB Polska Sp. z.o.o.

(57) **Abstract**

Invention relates to a mould for preparing non-self supporting soil sample for testing its mechanical properties. Mould comprises a mould base 6 having water inlet 9 and a pressure unit inlet 8 on which a lower sample base 10a that comprises a porous stone is placed, to said porous stone a filter paper and a latex membrane 4 are attached by means of O-rings 7a, wherein a base mount 3 surrounds a porous stone and a lower part of the water-soluble casing 1, while water-soluble casing 1 is placed between a latex membrane 4 and a temporary separable mould 5 on top of which a latex membrane 4 that contains the soil sample P is wrapped around, wherein on the sample P, an upper sample base is placed, that comprises in order: a filter paper, porous stone and on top of which an cover 10b with O-rings 7b is placed. Additionally the invention relates to a method of preparing non-self supporting soil samples for testing its mechanical properties, wherein a method comprises steps in which in the above described mould, that has an upper sample base and mould cover 10b detached in which: soil sample P is placed in the latex membrane by generating a vacuum by means of pressure unit attached to a mould base after all of the sample has been placed into a latex membrane, air is eliminated from the bottom sample base by introducing distilled water via water inlet and then the sample P is closed in a mould by putting upper sample base and cover on top of it separable mould is removed sample in the remaining of the mould is inserted into triaxial chamber water is introduced to the remaining of the mould via water inlet in such a manner that drainage of water is prevented, until soluble casing is dissolved, wherein during dissolving of a water-soluble casing, water escape from the pressure unit is prevented.

## Description

### Background of the invention

The invention is related to a mould for preparation of non-self supporting soil sample and a method of preparing a sample of non-self supporting soil for testing mechanical properties of soil. The invention is related to the technical field of geotechnical engineering.

### The state of the art

US20240102349A1 discloses a device for sampling soil for analysis. Device consists of a pipe for collecting soil (core) by inserting it into the soil and a transport tray for transporting a soil (core). Soil sample is collected manually, in the preferred embodiment it is collected mechanically. Once taken, the sample is pushed out of the tube directly onto the tray. This solution is however related only to collecting soil samples, not preparing it for testing mechanical properties.

AU2022201987A1 discloses a soil sample collection device comprising a laterally-undivided, tubular body divided longitudinally into distal and proximal parts; a thin, durable, extensively re-useable, metal liner to receive soil samples, said liner being easily loaded into and removed from said body; a thin, durable, extensively re-useable core catcher positioned adjacent the open, distal end of said body to restrict the outflow of said soil samples; a retainer positioned adjacent the open, distal end of said body and securing said metal liner and said core catcher in position within said body; a spacer sleeve locating said retainer; an interchangeable, ground-penetrating tip removably fixed to the open, distal end of said body; and an adaptor removably fixed to the proximal end of said body and by means of which said soil sample collection device is mounted upon deployment and drive means are employed to insert said device into, and withdraw it from, the ground. It is to be noted that AU2022201987A1 discloses only a device and method of collecting soil samples, not a method of preparing non-self supporting soil samples for testing its mechanical properties.

LU501984B1 discloses a multi-scale sample preparation device for a triaxial compression test of undisturbed soil, relating to the field of soil sample preparation. The multi- scale sample preparation device comprises a soil cutter frame and a base, wherein the soil cutter frame comprises a top plate, a bottom plate, and support columns, the support columns are uniformly fixed to a surface of the bottom plate in sequence, the tops of the support columns each are inserted into and fixed to the top plate, a screw is screwed into the surface of the bottom plate, the bottom of the base is provided with a screw hole, the base is fixed to an end of the screw by screwing the screw fitly into the screw hole, a soil sample fixing rod is slidably inserted in a surface of the top plate, the surface of the top plate is provided with a soil sample fixing rod limiter, and the bottom of the soil sample fixing rod is provided with a soil sample fixing plate. In the present invention, semicircular slits and a ring cutter structure significantly improve sample preparation efficiency and precision, and a cylinder sample with a relatively accurate size can be obtained. In addition, sample disturbance is reduced during cutting, which can effectively ensure sample quality. LU501984B1 does not relate to a preparation method for sampling non-self supporting soil for testing its mechanical properties. Additionally it does not disclose using a soluble cover for the preparation of samples to be tested and construction materials for the device according to LU501984B1 are different to the materials used in the construction according to the present invention.

CN114184498A discloses a method for evaluating the disturbance degree of an undisturbed hollow cylindrical sample of residual soil. The method comprises the following steps: a, taking a blocky sample and calculating the void ratio of an in-situ state soil sample; b, preparing an undisturbed hollow cylinder sample and loading the sample: cutting the undisturbed hollow cylinder sample, sleeving the sample with a rubber membrane, and mounting the sample on a hollow cylinder torsional shear apparatus; c, sample saturation: simultaneously increasing the inner confining pressure, the outer confining pressure and the back pressure from zero; d, sample consolidation: keeping the back pressure at 500kPa, gradually increasing the inner confining pressure and the outer confining pressure from 520kPa, and completing consolidation; e, calculating a disturbance index DI according to the drainage volume delta V, the sample size and the in-situ state soil sample void ratio in the consolidation process; f, evaluating the disturbance degree of the sample: evaluating the disturbance degree of the sample according to the DI value; g, sample shearing, unloading and instrument cleaning, wherein after evaluation is completed, the sample is the sample with small disturbance or extremely small disturbance. The method of CN114184498A is not suitable for preparing samples of non self-supporting soil and furthermore does not use the mold of the present invention.

RO130870B1 relates to an apparatus meant to determine the parameters of earth resistance to shear stress, applicable, in particular, in the field of geotechnics, and to a process for ensuring the shape of remanent earth samples. According to the invention, the apparatus comprises a piston (10) provided with two micro comparators (18), as well as with a dynamometer ring (12) and two cells (3) provided with two flexible membranes (7) for sealing purposes and with some mobile perforated pistons (6) to be brought into contact with the membrane (7) before the start of sample preparation and, further on, before the start of the test, to be withdrawn inside the cells (3) for allowing the sample deformation. The process, as claimed by the invention, provides for mounting, on a base plate (1), some plexiglass walls (2) and the cells (3) which are fixed by some angle bars (16), the position of the mobile perforated pistons (6) in contact with the rubber membranes (7) being secured by means of some worm presses (13), the walls (2) being then lubricated and the earth sample being prepared, after which the vertical load piston (10) and an upper metal plate (8) are mounted, followed by the mounting of a metal framework (14) and some tension members (15) in order to secure the position of pieces, a metal sabot (11) and the dynamometer ring (12) are placed between the press yoke and the rods of the piston (10), the micro comparators (18) are mounted onto the rods of the piston (10), pressurized water is introduced into the cells (3) and the position of the pistons (6) is maintained for 30 min, after which, by rotating a lever (5), they are withdrawn inside the cells (3) for allowing the free longitudinal deformation of samples. Device according to RO130870B1 does not use soluble casing and base on which the sample is placed is shaped differently. Method of forming the sample is also completely different, water is not used in the forming process.

CN 107462457 A discloses an electromagnetically controlled two-piece mould for soft soil sample loading and an experimental method. A wet soft sample can be loaded successively under the condition that the fixed shape of the wet soft sample is maintained and is not disturbed. The electromagnetically controlled two-piece mould for soft soil sample loading comprises two opposite mould pieces for preparing a cylindrical test sample, a lock and two water-permeable stones; two pairs of electromagnets which are controlled by an infrared switch remote controller are separately arranged at oppositely combined positions of the two opposite mould pieces; two spring holes for mounting springs are separately formed in middle positions of oppositely combined surfaces; the lock has an annular structure and is used for hooping a test sample; the inner diameter of the lock is equal to the outer diameters of the two opposite mould pieces; the lock is placed in the opposite mould pieces; and the two water-permeable stones are separately placed at the top ends and the bottom ends of the two opposite mould pieces. By the electromagnetically controlled two-piece mould for soft soil sample loading, a sample can be loaded in a triaxial apparatus under the condition that the fixed shape of the sample is maintained and is not disturbed, and a high-quality sample is provided for tests. Moreover, the electromagnetically controlled two-piece mould for soft soil sample loading is convenient to operate, short in consumed time and high in efficiency. The electromagnetically controlled two-piece mould for soft soil sample loading can also be manufactured on a large scale, and a large amount of time is saved. Construction of the mould according to CN107462457A is completely different compared to the mould of the present invention, which additionally does not comprise casing soluble in water.

CN102914459B discloses a hydraulic tri-axial sample preparation device and a sampling method thereof and belongs to the technical field of soil mass mechanics performance testing. The hydraulic tri-axial sample preparation device comprises a base, a hydraulic system is mounted on a door-shaped support which is mounted on the base, and a round protrusion is fixedly arranged at the centre of the base. The hydraulic system comprises a hydraulic applicator, a pressure transfer rod, a push head and a limiter, a sampling barrel is a cylindrical barrel and comprises a sample compacting barrel body, a sample discharging barrel body and a movable plug, and the inner diameter of the sample discharging barrel body is matched with the round protrusion of the base. The hydraulic tri-axial sample preparation device overcomes the defects that a traditional sample preparation device is incapable of compacting soil mass sufficiently and the density is non-uniform and can control the overall density of samples accurately during sample preparation to enable the samples to be more uniform, sample discharging is safe and rapid, and the method is simple and convenient. Document CN102914459B does not disclose the formation using a water-soluble casing and a rubber membrane to prepare samples of weak soils. This method also does not use water to prepare the sample.

CN102928267B relates to a parallel hydraulic triaxial sample preparation device and a sampling method, which belongs to the field of soil mechanical performance testing. The parallel hydraulic triaxial sample preparation device is composed of a base, a door-shaped bracket, a hydraulic system, a sampling cylinder and a counterforce pad. The door-shaped bracket is mounted on the base; the hydraulic system is mounted on the door-shaped bracket; a horizontal counterforce beam is mounted at the bottom of the door-shaped bracket; there is a sample discharge hole in the counterforce beam; the hydraulic system is composed of a hydraulic implementer, a pressure transfer rod, a pressing head, a plug and a horizontal beam; the sampling cylinder is a cylindrical hollow cylinder body; and the counterforce pad is composed of a counterforce cross plate, an upper side convexity and a lower side convexity. By using the parallel hydraulic triaxial sample preparation device, the disadvantage that the traditional sample preparation device cannot sufficiently compact the soil mass with uniform density is overcome; not only can the overall sample density and sample consistency be accurately controlled, but also 1-3 samples can be prepared at once with high efficiency; and the samples can be discharged conveniently. CN102928267B does not disclose the moulding using a water-soluble casing and a rubber membrane to prepare weak soil samples. This method also does not use water for sample preparation.

CN102901660B discloses a rotary rod type three-shaft sample preparation device and a sample preparation method thereof and belongs to the technical field of mechanical property testing of soil. The rotary rod type three-shaped sample preparation device comprises a base, wherein a door-shaped support is arranged on the base; a rotary rod piston is arranged on the door-shaped support; and two layers of circular bumps with unequal diameters are fixedly arranged in the centre of the base. A sample preparation barrel and a sample unloading barrel are cylindrical hollow barrel bodies; and the inner diameter of the sample preparation barrel and the inner diameter of the sample unloading barrel are respectively matched with the diameters of the two layers of circular bumps. By the sample preparation method, the shortcomings of the traditional sample preparation and sample unloading method are overcome, the total density of samples can be accurately controlled in a sample preparation process, the samples can be uniform, sample unloading is quick and safe, the method is simple and convenient, and the structure is simple. CN102901660B does not disclose preparation of non-self supporting soil sample in the mould comprising water soluble cover and rubber membrane. The method of CN102901660B also does not use water in the preparation of samples.

CN202837066U discloses a rotary rod type three-axis test sample preparation device and belongs to the field of a soil body mechanics performance testing technology. The rotary rod type three-axis test sample preparation device comprises a base; a door-shaped bracket is mounted on the base; a rotary rod piston is mounted on the door-shaped bracket; and two layers of circular bulges with different diameters are fixedly arranged at the centre of the base. A sample preparation cylinder and a sample unloading cylinder are cylindrical hollow cylinders; and the inner diameters of the sample preparation cylinder and the sample unloading cylinder are respectively adapted to the two layers of the circular bulges. According to a sample preparation method disclosed by the utility model, the defects of the traditional sample preparation and sample unloading method are overcome; the total density of a test sample can be accurately controlled in a sample preparation process and the test sample is more uniform; and the sample loading is fast and safe, the method is simple and convenient, and the structure is simple. CN202837066U does not disclose a device used for the preparation of non-self supporting soil sample in the mould comprising water soluble cover and rubber membrane. The method also does not use water in the preparation of samples.

SU909009A1 discloses a method for determining the stability of the contact of two different in size cohesive soils, including successive laying of coarse filter and fine-grained protected soils in the working chamber, applying a load to the surface of fine-grained soil, soaking the soils from bottom to top, creating water filtration from top to bottom, measuring the piezometric gradient of the filtration flow of precipitation of fine-grained soil, and its penetration depth, distinguishing it by the fact that in order to improve the accuracy of measurements, soluble material is placed between the coarse-grained filter and fine-grained soils in the process of laying soils, soaking soils is carried out until it is completely dissolved, and in the process of dissolution and filtration, the depth and amount of penetration of fine-grained soil are simultaneously measured into the pores of coarse soil. Method of SU909009A1 is used to examine a stability of the contact of two different in size cohesive soils, not mechanical properties of the soil. Additionally, the invention of SU909009A1 does not mention using casing made of PVA or a rubber membrane.

### Purpose of the invention

Soft soils, including marine clays, sediments from dredging, volatile ash pulp and organic soils are currently a challenge in the geotechnical projects. These soils are often used in the projects of marine wind turbines (Harte, M., Basu, B. & Nielsen, S., 2012. Dynamic analysis of wind turbines including soil-structure interaction. Engineering Structures, Volume 45, pp. 509-518; Wood, T., 2016. PhD Thesis: On the Small Strain Stiffness of Some Scandinavian Soft Clays and Impact on Deep Excavations. Gothenburg, Sweden: Department of Civil and Environmental Engineering Chalmers University of Technology) are characterized by a high moisture content, what makes them fragile in terms of transporting and testing (Hight, D. W, 2003. Sampling Effects in Soft Clay: An Update on Ladd and Lambe (1963). s.l., s.n.; Lunne, T. et al., 2006. Effects of sample disturbance and consolidation procedures on measured shear strength of soft marine Norwegian clays. Canadian Geotechnical Journal, 43(7); Karlsrud, K. & Hernandez-Martinez, F. G., 2013. Strength and deformation properties of Norwegian clays from laboratory tests on high-quality block samples. Canadian Geotechnical Journal, 50(12).; Karlsson, M., Emdal, A. & Dijkstra, J., 2016. Consequences of sample disturbance when predicting long-term settlements in soft clay. Canadian Geotechnical Journal, 53(12).; Balachowski, L., Mi 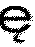 dlarz, K. & Konkol, J., 2019. Geotechnical characterization of soft soil deposits in Northern Poland. Engineering Geology, Volume 259.).

Proper sampling and quality assessment are crucial for correct measurement results. Due to low effective stress and no load on the soil, it is difficult to maintain its shape, which complicates laboratory tests such as triaxial tests. Their deformability and low weight make the preparation, consolidation and shearing stages even more difficult. Determination of consolidation parameters and strength in soft soils often involves the oedometer/consolidometer method, the vane test or the falling cone test (Seng, S. & Tanaka, H., 2012. Properties of very soft clays: A study of thixotropic hardening and behavior under low consolidation pressure. Soils and Foundations, 52(2), pp. 335-345). However, these methods do not fully assess the consolidation, shear and stress-strain properties of the soil at different strain levels. Conventional triaxial tests can investigate these parameters simultaneously but are inappropriate for soils that are unable to support their own weight.

Therefore, it is advisable to develop a triaxial testing method that is cost-effective and easy-to implement Previous attempts using split moulds required significant modifications and skilled operators (Wei, Z. et al., 2014. Improved Isotropically Consolidated Undrained Triaxial Test Method for Non Self-Supporting Materials. Geotechnical Testing Journal). For weak soils, traditional methods involving negative pressure after mould removal are unsuitable. Therefore, new solutions are essential for testing these soils effectively. To serve this purpose, a method for producing weak soil specimens using a "lost mould" made from water-soluble PVA, preferably 3D printed from the PVA filament, has been developed. It is to be noted that other forming techniques such as injection moulding can be used to manufacture the water-soluble casing of the present invention. A sample of the soil that cannot support itself in the dimensions that are required by the testing procedure (a cylinder having a height that is twice the diameter) is placed in the latex membrane that is inserted into water-soluble casing. Water-soluble casing is dissolved in the triaxial chamber under low water pressure, leaving cylindrical sample of the soil that is surrounded by a latex membrane. It is very essential to prevent water escaping from the pressure unit during dissolving of a water-soluble casing, since such escape may lead to sample deformation which is very undesirable in terms of the accuracy of the testing procedure. Subsequently after the casing is dissolved, tests such as sand identification, saturation, consolidation, and shearing can be performed.

### Essence of the invention

Essence of the invention is the mould for preparing a sample of non-self supporting soil for testing its mechanical properties, wherein said mould comprises: a mould base 6 having water inlet 9 and a pressure unit inlet 8 on which a lower sample base 10a that comprises a porous stone is placed, to said porous stone a filter paper and a latex membrane 4 are attached by means of O-rings 7a, wherein a base mount 3 surrounds a porous stone and a lower part of the water-soluble casing 1, while water-soluble casing 1 is placed between a latex membrane 4 and a temporary separable mould 5 on top of which a latex membrane 4 that contains the soil sample P is wrapped around, wherein on the sample P, an upper sample base is placed, that comprises in order: a filter paper, porous stone and on top of which an cover 10b with O-rings 1b is placed wherein during dissolution of the soluble cover water is prevented from escaping through the pressure system.

Preferably, water-soluble casing is made of polyvinyl alcohol (PVA).

Preferably, a water-soluble casing and a base-mount are manufactured by 3D-printing.

Preferably, a base-mount is made of polylactic acid or acrylonitrile-butadiene-styrene copolymer.

Preferably, a tension force sensor is attached to a cover.

Preferably a base-mount 3 that surrounds a porous stone is annular shaped.

Preferably the casing 1 is 75 mm tall and has a 38 mm of inner diameter and 0.4 mm of thickness.

The present invention is also related to a method of preparing non-self supporting soil samples for testing its mechanical properties, wherein a method comprises steps in which in the above described mould, that has an upper sample base and mould cover detached in which:
- soil sample P is placed in the latex membrane 4 by generating a vacuum by means of pressure unit 8 attached to a mould base 6
- after all of the sample has been placed into a latex membrane, air is eliminated from the bottom sample base 10a by introducing distilled water via water inlet 9 and then
- the sample P is closed in a mould by putting upper sample base 10b and cover 2 with O-rings 7b on top of it
- separable mould 5 is removed
- sample in the remaining of the mould is inserted into triaxial chamber
- water is introduced to the remaining of the mould via water inlet in such a manner that drainage of water is prevented, until soluble casing is dissolved.

Preferably testing consists of sand identification, saturation, consolidation, and shearing of the sample.

Preferably dissolving of the soluble casing takes three hours.

Preferably vacuum value during inserting a soil sample into the mould is 10kPa.

Preferably soil sample is placed into the mould by suction.

Preferably, tension force sensor is detached prior to suction of the soil sample into the cover.

Preferably, if the soil is dry sand, soil sample if formed by pouring it into the mould in multiple, compacted layers.

The mould 75 mm tall with a 38 mm inner diameter and 0.4 mm thickness, takes about 1.5 hours to print. In the triaxial chamber, the mould dissolves under low water pressure, leaving a cylindrical soil specimen surrounded by a latex membrane. It is crucial to prevent water from escaping through the pressure system during dissolution. After mould dissolution, saturation, consolidation, and shearing can be performed.

### Brief description of the drawings

Fig. 1 presents mould with soil sample inside.
Fig. 2 presents scheme of forming soil sample, that is divided into two types of samples: I- soft and wet sample, II - sample in the form of dry sand.
Fig. 3 presents a mould that is ready-to-use.
Fig. 4 presents a process of dissolving a soluble casing in triaxial chamber in three time milestones after one, three and fourth hour.
Fig. 5 presents two kinds of sample forming methods a) conventional- without using a soluble casing and b) a method using said casing.
Fig. 6 presents graphs illustrating a relation between stress deviator and axial strain (a) and relation between volumetric strain and axial strain (b); for dense (80% relative density) and medium dense (40% relative density) sands.
Fig. 7 presents graphs that illustrate kaolin specimen responses to consolidation stresses, showing water flow (a) and consolidation time and volumetric strain (b).
Fig. 8 presents graphs that illustrate a relation between stress deviator (a) and changes in water pressure within the soil pores (b) throughout the course of the tests.

In the drawing following numerical markings were used: 1- soluble casing; 2- mould cover; 3- base mount; 4- latex membrane; 5- temporary separable mould; 6- base; 7a, 7b- O-rings; 8- pressure unit; 9- water inlet; 10a- bottom sample base; 10b- upper sample base; P- soil sample;

### Detailed description of the invention

The invention relates to a mould for forming a soil sample for testing its mechanical properties as well as a method of forming the soil sample. Mould according to the invention can be used to form, dust, clay and silt, among others.

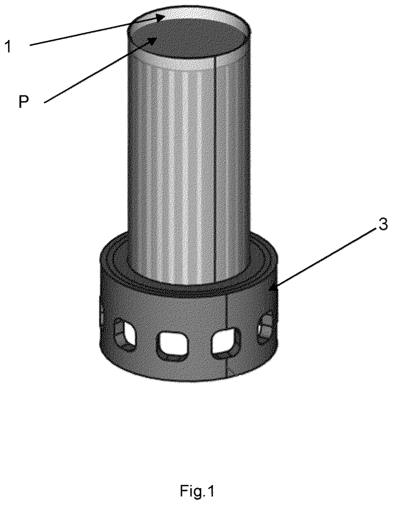

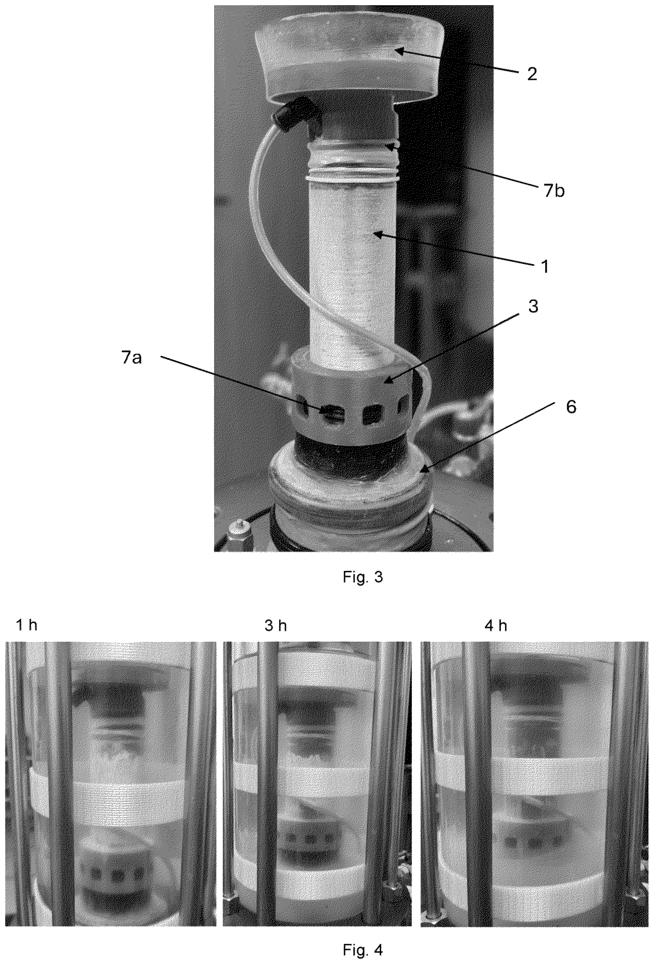

A mould for preparing non-self supporting soil sample for testing its mechanical properties comprises a mould base 6 having water inlet 9 and a pressure unit inlet 8 on which a lower sample base 10a that comprises a porous stone (not shown for the clarity of the drawing), that is preferably made of sintered brass or corundum (materials typically used in geotechnics) is placed, to said porous stone a filter paper (not shown for the clarity of the drawing), and a latex membrane 4 are attached by means of O-rings 7a. O-rings 7a and 7b serve sealing purpose by holding a latex membrane 4 to the cover 2 and base 6. Thickness and mechanical properties of filter paper and latex membrane are defined in ISO- 7892-09. A base mount 3 is placed on the mould base 6 and it surrounds a porous stone and a lower part of the water-soluble casing 1 of the soil sample P. Base mount 3 is preferably ring-shaped. Water-soluble casing 1 is placed between a latex membrane 4 and a temporary separable mould 5 on top of which a latex membrane 4 that contains the soil sample P (see Fig. 1) is wrapped around. Temporary separable mould 5 serves a purpose of stabilising and supporting a soluble casing 1, protecting it from mechanical damage during sucking a soil sample P into the mould. On the sample P, an upper sample base 10b is placed. As shown in Fig. 3 upper sample base 10b comprises in order: a filter paper, porous stone and a cover 10b with O-rings 7b and sensor (not shown) that allows axial movement during removing a sample from the mould to maintain it in the axis of the device.

Soluble casing 1 and base mount 3 are manufactured by 3D printing, wherein the casing 1 is made of polyvinyl alcohol, while base mount 3 is made of polylactic acid or acrylonitrile-butadiene-styrene copolymer. Printing a mould having a height of 75mm, internal diameter of 38 mm and a thickness of 0,4 mm takes approximately 1,5 hours.

Invention also relates to a method of preparing non-self supporting soil samples for testing mechanical properties of the soil.

Examples of soil that can be tested using a mould of the present invention include kaolin and sand.

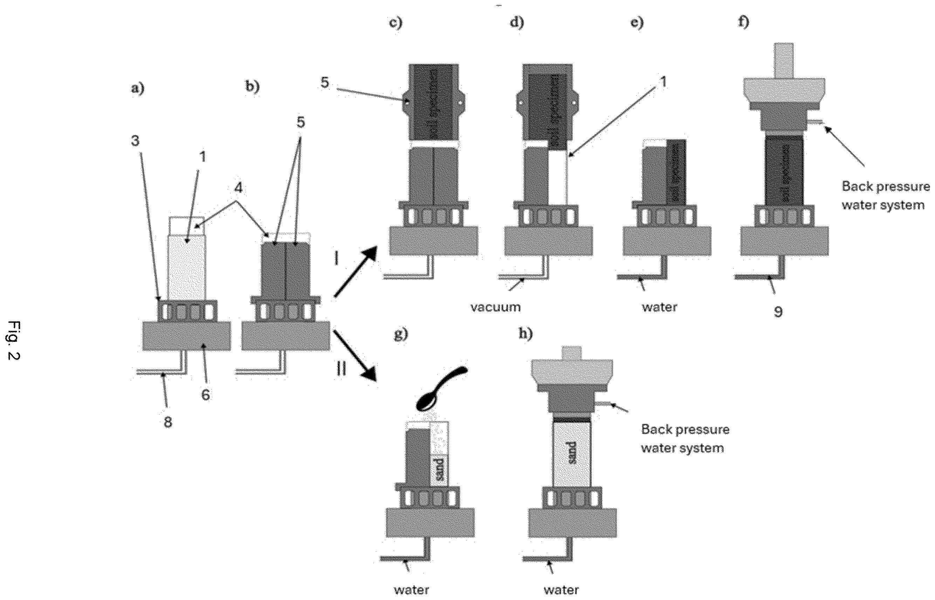

In the first step, to the above described mould that has an upper sample base 2 and mould cover 10b detached (as shown in Fig. 1 p. 1a), separable mould 5 is attached (as shown in Fig. 2, p. b)) and subsequently a soil sample P is placed in the latex membrane 4 of the above described mould by generating a vacuum of 5 to 25 kPa by means of pressure unit 8 attached to a mould base 6, which causes sucking a soil sample into the latex membrane 4 (as shown in Fig. 2, p. c) and d)). In the preferred embodiment a tension force sensor is detached from the mould base 6 prior to sucking a sample P into a soluble casing 1, to prevent its damage. Subsequently, after all of the sample P has been placed into a latex membrane 4, air is eliminated from the bottom sample base 10a by introducing distilled deaerated water via water inlet 9 (see Fig. 2 p. e)). It is recognized that the bottom sample base 10a is deaerated if pressure increases immediately after introducing the water into the system. In the next step the sample P is closed in a mould by putting upper sample base 10b and cover 2 with O-rings 7b on top of it, then a separable mould 5 is removed as shown in the Fig. 2 p. f). Sample P in the remaining of the mould is inserted into triaxial chamber, into which water is introduced via water inlet in such a manner that drainage of water is prevented, until soluble casing 1 is dissolved. A mould inserted into the triaxial chamber and a process of solving a soluble casing 1 are shown in Fig. 4. A process of solving soluble casing takes 2-4 hours, preferably 3 hours.

If a non-self supporting soil sample is dry sand, the sample is formed by pouring it into the mould in multiple, compacted layers (see Fig. 2, II).

### Example

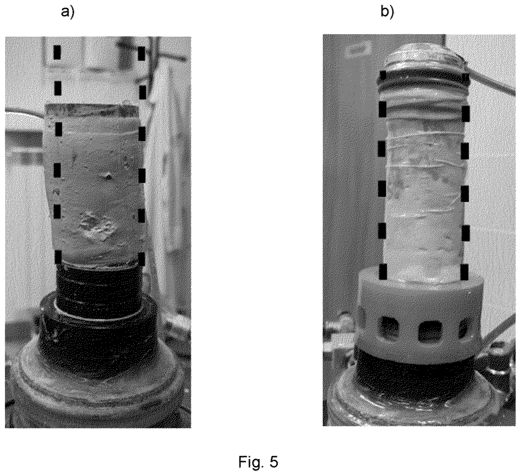

Sample of kaolin were formed using two different forming methods: conventional (Fig. 5a) and a method according to the present invention (Fig. 5b). Fig. 5a shows a sample of kaolin extracted from the casing, showing dimensions and shape loss due to high moisture content, which caused that performing tests was impossible. On the other hand, Fig. 5g shows a sample formed by the above mentioned method according to the present invention, which was consolidated to effective tension of 100 kPa while maintaining its independence, what made the test easier.

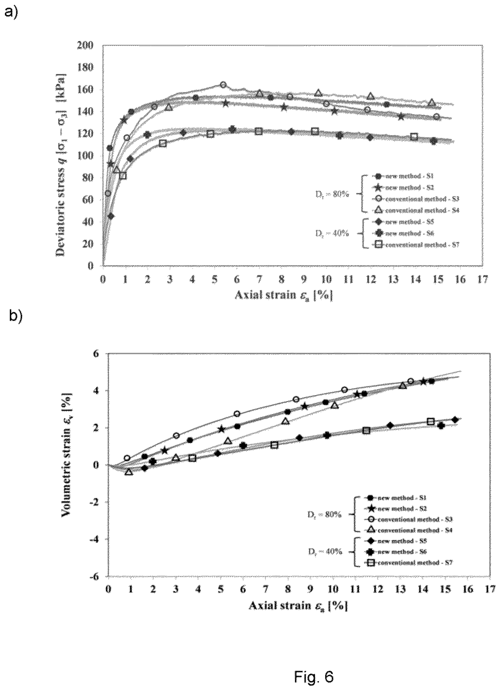

Fig. 6 shows results of dense sand (80% relative density) and medium dense sand (40% relative density). Dense sand showed work hardening with drainage, achieving maximum with deformation at the level of 4-5% for samples formed according to the present invention (Fig. 6a). Medium dense sand showed similar behaviour but having lower stress deviator values (Fig. 6a).

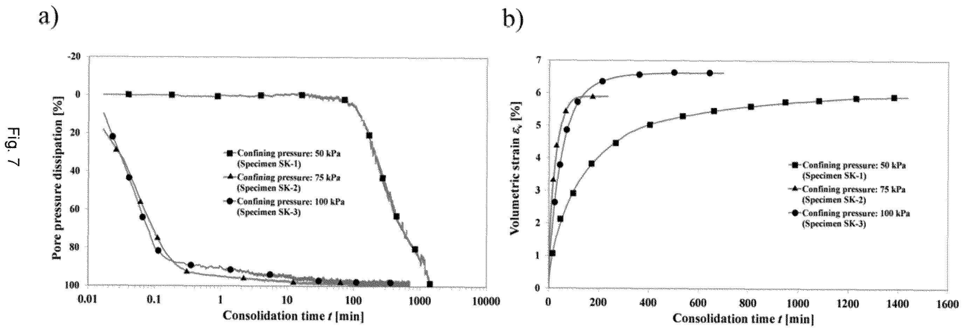

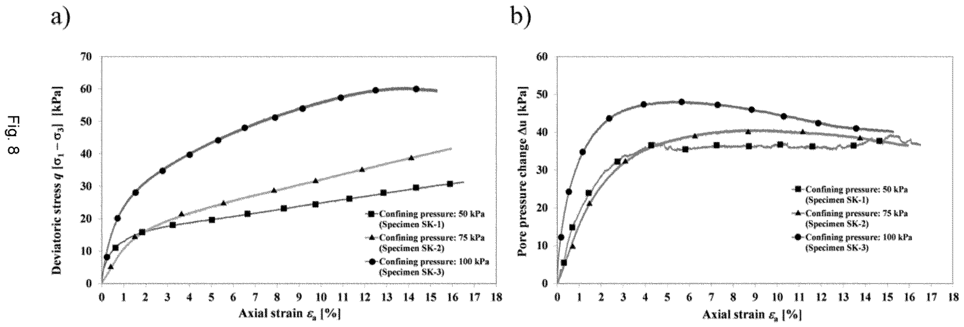

Fig. 7 shows kaolin specimen responses to consolidation stresses, showing water flow and consolidation time and volumetric strain (Fig. 7a). Sample SK-2 required twice as long consolidation time (Fig. 7b) (one-way drainage). Results of the shear test conducted in no-drain conditions are shown in Fig. 8. Test results show increased stress deviations in samples SK-1 and SK-2, showing constant increase until the end of the test (Fig. 8a). However, in sample SK-3 maximum value of said value was obtained when axially deformed by 13,5%, as shown in Fig. 8a. Fig. 8b shows changes of the water pressure in the soil pores during the test. Results of shear test show typical reaction for the soils with a predominance of loamy fractions that has important impact on mineral content (Ladd & Foot, 1974; Lade, 2016).

Method of forming soil sample according to the present invention requires minimum hardware modification, mainly by using plastic 3D printed parts. Reduction of costs can be achieved by making a form from commercially available materials. Moreover, said method allows to independently maintain the shape of the soil, preventing water flow during dissolving of the casing and maintaining stress and strain conditions. Tests conducted on the samples that cannot stand by themselves, meet the requirements of triaxial tests.

Samples of high and medium density sands behave typically under consolidation pressure. Method can be used to test stress-strain characteristics of the sand under triaxial compression. Above described method greatly simplifies process of testing non-self supporting soils.

To evaluate practicability of the method according to the present invention it is required to test larger samples, which may require using forms having bigger wall thickness, for example 0,2 to 1 mm, preferably 0.4 mm of thickness.

In the context of the present invention, non-self supporting soil is a loose type of substrate, the fragments of which easily crumble in the hands. They are defined as mineral or organic, not meeting the conditions for mineral soil. Among the types of non-self supporting soil, gravel, coarse sand, medium sand, as well as fine-grained and silty sand, are distinguished.

## Claims

1. Mould for preparing a sample of non-self supporting soil for testing its mechanical properties, **characterized in that** it comprises:
- a mould base (6) having water inlet (9) and a pressure unit inlet (8) on which
- a lower sample base (10a) that comprises a porous stone is placed, to said porous stone a filter paper and a latex membrane (4) are attached by means of O-rings (7a),
- wherein a base mount (3) surrounds a porous stone and a lower part of the water-soluble casing (1), while
- water-soluble casing (1) is placed between a latex membrane (4) and a temporary separable mould (5) on top of which a latex membrane (4) that contains the soil sample (P) is wrapped around,
- wherein on the sample (P), an upper sample base is placed, that comprises in order: a filter paper, porous stone and on top of which an cover (2) with O-rings (7b) is placed, wherein during dissolution of the soluble cover water is prevented from escaping through the pressure system.

2. Mould according to claim 1, wherein water-soluble casing is made of polyvinyl alcohol.

3. Mould according to claims 1 or 2, wherein a water-soluble casing and a base-mount are manufactured by 3D-printing.

4. Mould according to any of the preceding claims, wherein a base-mount is made of polylactic acid or acrylonitrile-butadiene-styrene copolymer.

5. Mould according to any of the preceding claims, wherein a tension force sensor is attached to a cover.

6. Mould according to any of the preceding claims, wherein a base-mount (3) that surrounds a porous stone is annular shaped.

7. Mould according to any of the preceding claims, wherein the casing (1) is 75 mm tall and has a 38 mm of inner diameter and 0,2 to 1 mm, preferably 0.4 mm of thickness.

8. Method of preparing non-self supporting soil samples for testing its mechanical properties, wherein a method comprises steps in which in the above described mould, that has an upper sample base (10b) and mould cover (2) detached in which:
- soil sample (P) is placed in the latex membrane (4) by generating a vacuum by means of pressure unit (8) attached to a mould base (6),
- after all of the sample has been placed into a latex membrane, air is eliminated from the bottom sample base (10a) by introducing distilled water via water inlet (9) and then
- the sample (P) is closed in a mould according to claims 1-7, by putting upper sample base (10b) and cover (2) on top of it
- separable mould (5) is removed
- sample (P) in the remaining of the mould is inserted into triaxial chamber
- water is introduced to the remaining of the mould via water inlet in such a manner that drainage of water is prevented, until soluble casing is dissolved, wherein during dissolving of a water-soluble casing, water escape from the pressure unit is prevented.

9. Method according to claim 8, wherein testing consists of sand identification, saturation, consolidation, and shearing of the sample.

10. Method according to claims 8-9, wherein dissolving of the soluble casing takes three hours.

11. Method according to claims 8-10, wherein vacuum value during inserting a soil sample into the mould is 10kPa.

12. Method according to claims 8-11, wherein soil sample is placed into the mould by suction.

13. Method according to claims 8-12, wherein tension force sensor is detached prior to suction of the soil sample (P) into the cover (1).

14. Method according to claims 8-12, wherein the soil is dry sand, soil sample is formed by pouring it into the mould in multiple, compacted layers.
